Europäisches Patentamt

(19) European Patent Office

Office européen des brevets

(11) Publication number: **0 085 805**
**B1**

(12) **EUROPEAN PATENT SPECIFICATION**

(45) Date of publication of patent specification: **12.03.86**

(21) Application number: **82306059.5**

(22) Date of filing: **15.11.82**

(51) Int. Cl.⁴: **C 12 N 1/00, B 41 M 5/00, B 01 J 13/02**

(54) Process for encapsulating substances by means of microorganisms, and the encapsulated products obtained thereby.

(30) Priority: **21.11.81 GB 8135153**

(43) Date of publication of application:
**17.08.83 Bulletin 83/33**

(45) Publication of the grant of the patent:
**12.03.86 Bulletin 86/11**

(84) Designated Contracting States:
**AT BE CH DE FR GB IT LI LU NL SE**

(56) References cited:
**FR-A-2 179 528**
**US-H- 498 208**

(73) Proprietor: **DUNLOP LIMITED**
**Dunlop House Ryder Street St. James's**
**London SW1Y 6PX (GB)**

(72) Inventor: **German, Reginald James**
**Greenoaks Woodlands Drive**
**Sunbury-on-Thames Middlesex (GB)**
Inventor: **Morris, Gwilym Gareth**
**102 Clarence Road Four Oaks**
**Sutton Coldfield West Midlands (GB)**
Inventor: **Pannell, Nahida Abid**
**55, Grestone Avenue Handsworth Wood**
**Birmingham B20 1AU West Midlands (GB)**

(74) Representative: **Sparrow, Alvar Alfred et al**
**Dunlop Limited Group Patent Department P.O. Box 504**
**Erdington, Birmingham B24 9QH (GB)**

The file contains technical information submitted after the application was filed and not included in this specification

Courier Press, Leamington Spa, England.

## Description

This invention relates to a method of encapsulation and to encapsulated products involving a microbial capsule.

The encapsulation of various substances for protection or to delay reactivity is well-known.

In U.S. Patent Specification 4,001,480 (and the corresponding U.S. Published Patent Application B498,208) it has been proposed to make encapsulated products by contacting fat-soluble substances with fungi having a very high natural fat content. According to that patent specification the substances must be soluble in the natural fat, or lipid, of the fungi and the fungi must have a natural fat content of about 40—60% by weight. Thus, for instance, if it is desired to encapsulate a dye within the fungus, the dye must be soluble in the natural fat of the fungus.

In French Patent Specification 2,179,528 there is described a method of causing certain materials to be absorbed and/or fixed by microbes, in which a microbe such as pressed industrial yeast is treated with a plasmolyser, i.e. a substance which causes contraction or shrinking of the microbial cytoplasm by exosmosis of water, and then an aqueous solution of a material such as neodymium chloride, magnesium chloride or onion juice is added under certain conditions so that the aqueous material is absorbed in place of the extracted cytoplasmic water.

It is now found that it is possible to encapsulate materials satisfactorily in a microbe having a natural fat, i.e. lipid, content of less than 40% by weight, without the use of a plasmolyser. This is achieved by taking a microbe having a low lipid content, and treating it with (i) a lipid-extending substance which will be taken into the microbe and be retained passively therein and (ii) a material which is soluble or dispersible in the lipid-extending substance so that the material is absorbed by the microbe. It is also found that this technique enables the encapsulation of a material which is insoluble in the lipid of the microbe provided that it is soluble or dispersible in the lipid-extending substance. Further, it is possible also to apply this technique to microbes having lipid contents of 40% or more.

Therefore, according to the present invention, there is provided a method of producing an encapsulated material which comprises treating a grown microbe with a material to be encapsulated, characterised in that the microbe is contacted with an organic lipid-extending substance being an organic liquid which is capable of being taken up by the microbe so that it is retained passively therein and which is a solvent or microdispersing medium for the material to be encapsulated, and simultaneously and/or subsequently contacting the microbe with the material to be encapsulated, said material being employed as a solution or microdispersion in the organic lipid-extending substance or as a solution or microdispersion in a further organic lipid-extending substance or in a liquid which is miscible with the first-mentioned lipid-extending substance, so that both the organic lipid-extending substance and the material are taken into and retained passively in the microbe.

According to the present invention also there are provided encapsulated materials having the features resulting from the method defined herein.

By the term "grown microbe" there is meant a microbe which has been harvested from its culture medium. The microbe may have a lipid content of, for example, up to 35% by weight, although microbes having higher lipid contents may also be treated in accordance with this invention. A preferred microbe is a fungus, particularly a yeast. If necessary, the lipid content of a microbe may be increased by cultivation under reduced-nitrogen conditions. Examples of suitable microbes are *Lipomyces* species such as *lipofer* and *starkeyi*, *Trichosporon* species such as *pullulans* and *cutaneum*, and *Candida* species such as *curvata* and *107*. If desired, a microbe which is not normally oleaginous, such as *Saccharomyces cerevisiae* (bakers yeast) or *Candida utilis*, may be employed.

Preferably the microbe has a large cell size, e.g. of 5 to 20 μm diameter.

The lipid-extending substance is a substance which is miscible with the microbial lipid and which is capable of entering the microbe by diffusion through the cell wall to extend or swell the lipid without cell rupture. Usually it will be a liquid at normal temperatures but it is possible to employ a substance which is a solid at normal temperatures provided that it can be liquified at slightly elevated temperatures which may be employed in the lipid-extension process.

Suitable lipid-extending substances may be selected by the following tests.

A. The substance may be mixed with an equal quantity of a microbe in the form of an aqueous slurry (e.g. 1 g of substance and 1 g of microbe as a 20% aqueous slurry) for 2 to 6 hours at 30 to 50°C, the degree of mixing being sufficient to maintain homogeneity of the mixture (e.g. by use of a standard laboratory hot plate magnetic stirrer at medium speed). The microbe is then harvested, suitably by centrifugation (e.g. at about 2000 rpm for 15 minutes), and the harvested microbe is examined under a microscope. Suitable substances are shown by the presence of a large glistening globule in the cytoplasm. Usually the globule will occupy substantially all of the cytoplasm volume.

B. The substance and microbe are mixed and the resultant microbe is harvested, as in A, and then the microbe is analysed qualitatively and/or quantitatively such as by treatment with a solvent for the substance (e.g. methanol or methanol/water (e.g. 80/20 v/v)) and analysis of the extract by a chromatographic technique such as gas-liquid chromatography to detect the presence of the substance.

C. The production of the mixture as in A is modified by including a known amount (e.g. 0.02 g) of a dye or other colourant in the substance, the harvested microbe is solvent-extracted as in B and the extract is examined for colour such as by use of a spectrophotometer. If the dye is a leuco dye the extract should be treated with an acid prior to photometric examination.

D. The production of the mixture as in A is modified by including a known amount (e.g. 0.02 g) of a dye in the substance, the harvested microbe is made into an aqueous suspension (e.g. 20% dry weight of microbe), the suspension is applied to the surface of a sheet of paper at a density of about 3 to 10 g.m$^{-2}$ (e.g. by means of a doctor blade), the paper is air-dried, and then the paper is subjected to localised pressure sufficient to rupture the microbe (e.g. by use of a typewriter or manual writing instrument) to see if the dye is emitted from the ruptured microbe. Suitably the dye may be a leuco dye, e.g. Crystal Violet Lactone, the microbe-coated paper surface may be assembled in contact with the clay-coated surface of another paper sheet, and the assembly subjected to the localised pressure.

E. The substance is mixed with an equal quantity (e.g. 1 ml) of soybean oil (e.g. by shaking in a test-tube at room temperature). Suitable substances are those which are seen to be miscible with soybean oil.

The choice of lipid-extending substance appears to be independent of the microbe to be treated.

One group of substances consists of alcohols having more than three carbon atoms, especially aliphatic alcohols. The alcohols may have one or more hydroxyl groups. Examples of suitable alcohols are primary alcohols, especially those having 4 to 15 carbon atoms, e.g. n-butanol, octanol, decanol and mixtures of long-chain primary alcohols, mainly of 9 to 15 carbon atoms; secondary alcohols e.g. iso-butanol and octan-2-ol; tertiary alcohols e.g. tertiary-butanol; and diethylene glycol (O(CH$_2$CH$_2$OH)$_2$).

Another group of suitable substances consists of esters, especially those in which the alcohol component has more than two carbon atoms. The esters may be derived from acids having one or more carboxylic groups, for example 2 or 3 carboxylic groups, and from alcohols having one or more hydroxyl groups, for example 2 or 3 hydroxyl groups. The acid and alcohol components may be aromatic or aliphatic. The acid component may be a hydroxycarboxylic acid. Examples of suitable esters are 2-ethylhexyl acetate, di-2-ethylhexyl adipate, di-iso-butyl phthalate, butyl benzyl phthalate, acetyl tributyl citrate, 2,2,4-trimethyl-1,3-pentanediol iso-butyrate, glyceryl triacetate (triacetin) and glyceryl tributyrate.

Other suitable substances are some liquid hydrocarbons, for example aromatic hydrocarbons such as xylene, and hydrogenated aromatic hydrocarbons such as that available as "Santosol 340" and the mixture of 40% hydrogenated terphenyls available as "HB 40".

Mixtures of lipid-extending substances may be employed.

In some cases, particularly when the substance is normally viscous, it may be desirable to thin or dilute the substance with a thinner such as acetone in order to facilitate the rate of absorption by the microbe and/or the amount of substance absorbed. Substances which may benefit from such thinning include "Santosol 340", "HB 40" and the commercially available mixtures of long-chain primary alcohols.

When a material is to be encapsulated in accordance with the invention, the particular lipid-extending substance employed should be a solvent or dispersant for the material. The encapsulatable material might not be soluble in the microbial lipid; however, solubility or compatibility with the lipid is preferred in order to enable as such as possible of the material to be encapsulated. The material may be a solid or a liquid.

The encapsulation method is particularly suitable for producing encapsulated dyes, for example leuco dyes. Examples of suitable dyes are Sudan Green, Sudan Blue, Ethyl Eosin, Crystal Violet Lactone, Methylene Blue Lactone, Malachite Green Lactone and Benzoyl Leuco Methylene Blue. Other examples of encapsulatable materials are adhesives, adhesive components, pharmaceuticals (e.g. stimulants such as pherones), flavours rodenticides, insecticides, herbicides, fungicides and odiferous materials. Two or more reactive components may be encapsulated by separate cells, for example the reactive components of a two-component adhesive.

Conveniently the material is dissolved or microdispersed in the lipid-extending substance before contact with the microbe. Alternatively or additionally, the microbial lipid may be extended by the lipid-extending substance and then the microbe may be treated with the encapsulatable material. In the latter case the material may be in the form of a solution or dispersion in further lipid-extending substance or in a liquid which is miscible with the lipid-extending substance.

When the microbe is treated with the lipid-extending substance, it should be intact, i.e. not lysed. Usually the treatment will comprise stirring the microbe in the lipid-extending substance. The microbe may be in the form of a paste or slurry in water but a preferred technique comprises employing the microbe in a merely moist form. Conveniently the treatment may be performed at room temperature or at a slightly elevated temperature, e.g. 40 to 50°C, which in some cases may increase microbial absorption of the substance. An elevated temperature may also increase the amount of encapsulatable material dissolved in the lipid-extending substance. Usually the treatment time should be at least 2 hours, more usually at least 3 hours, to achieve optimum lipid-extension.

The amount of material encapsulated will depend on the extended lipid content of the microbe and on the solubility or dispersibility of the material therein. In order to attain encapsulation of a large quantity of material and to facilitate spreading or dispersal of the material if and when subsequently released from the microbial capsules, usually it is desirable for a large amount of lipid-extending substance to be taken up by the microbe. In most cases it is not necessary for the amount of lipid-extending substance employed to exceed the weight of the microbe.

The encapsulated material may find application as a free-standing product or adhered to a substrate. An example of a use of this invention is in the production of "carbonless" copy paper in which a coating of capsules containing a dye is adhered to one side of a sheet of paper, so that when the paper is subjected to pressure, e.g. by the type-face of a typewriter or by a manual writing implement, the print will be duplicated on a paper sheet in contact with the coating. It is found when encapsulated dyes produced according to the present invention are applied in the form of an aqueous suspension to the paper, the capsules adhere to the paper satisfactorily without the assistance of a binder or adhesive. The encapsulated material may be a material which produces a strong colour only when it reacts with another material, which may itself be encapsulated or may be in the form of a non-encapsulated coating on the paper. For instance, the encapsulated material may be a leuco dye which is oxidisable by an acidic material such as an acidic clay to produce the desired print colour. It is preferred that one side of the paper sheet has a coating of the encapsulated material and the other side has an acidic clay coating, so that several sheets can be assembled with the clay-coated face of one sheet in contact with the capsule-coated face of the adjacent sheet. Alternatively a mixed coating of capsules and clay may be employed.

In some cases it may be desirable to employ more than one encapsulated dye in order to attain a desired colour.

An alternative use of capsules of this invention is in conditions such that the release of the encapsulated material is delayed or prolonged by a slow or gradual lysis of the microbial cells. This could be advantageous for the administration of pharmaceuticals.

The invention is illustrated in the following Examples.

Example I

*Lipomyces lipofer* 5841 of Central Bureau voor Schimmelcultures (CBS) was grown in the following medium

|  | g.l$^{-1}$ |
|---|---|
| glucose | 40 |
| ammonium tartrate | 3 |
| potassium dihydrogen orthophosphate | 5 |
| magnesium sulphate 7H$_2$O | 0.2 |
| sodium chloride | 0.1 |
| calcium chloride 2H$_2$O | 0.01 |
| yeast extract powder | 1 |
| ferric chloride | 0.002 |

The culture was grown in shake-flasks using an orbital incubator at 180 rpm and 28°C for 72 hours. The grown yeast cells were harvested by centrifugation (800 rpm for 5 minutes).

The lipid content of the yeast was determined by the following procedure. Approximately 1.5 g of wet cells (0.2 g dry weight) were weighed accurately and then hydrolysed with 2 ml of 6N HCl in a boiling water bath for 2 hours. The hydrolysate was mixed with 30 ml of a 2:1 (v/v) chloroform:methanol mixture overnight. The resulting mixture was transferred to a separating funnel with 5 ml of 0.9% aqueous NaCl and the chloroform/methanol layer was separated. The remaining mixture was washed twice with 10 ml of chloroform, and the washings were combined and evaporated to dryness in a pre-weighed beaker. The beaker was then re-weighed to determine the weight of the lipid, which was found to be about 30% by weight based on the dry weight of the yeast.

Yeast produced from the above culture was made into a 15% (w/v) slurry with distilled water, and a known weight of this aqueous slurry, containing 1 g (dry weight) of microbe, was mixed with 0.8 g of a 1% (w/v) solution of crystal violet lactone in 2-ethylhexyl acetate. The mixture was stirred for 6 hours at room temperature using a standard laboratory hot plate magnetic stirrer at medium speed to maintain homogeneity and the resulting microbial encapsulated dye was harvested by centrifugation. The harvested capsules were suspended in distilled water at a concentration of approximately 15% dry weight of cells in the water and the suspension was applied to the opposite side of clay-coated paper using K-Bar No. 4 supplied by R. K. Print-Coat Instruments Ltd., U.K. The quantity of capsules applied to the paper was of the order of 6—10 g.m$^{-2}$. The paper was dried and tested for duplication ability in a standard office typewriter.

It was found to function satisfactorily as a "carbonless" copy paper.

Example II

The procedure of Example I was repeated except that, instead of *Lipomyces lipofer* 5841, the following microbes were employed individually. The lipid contents of the microbes are given below as approximate percentages by weight based on the dry weight of the microbe.

|  | % lipid |
|---|---|
| *Lipomyces lipofer* 944 (NCYC) | 60 |
| *Lipomyces lipofer* 5842 (CBS) | 40 |
| *Lipomyces lipofer* 982 (NCYC) | 60 |
| *Lipomyces starkeyi* 1809 (CBS) | 35 |
| *Trichosporon cutaneum* 40 (IOWA) | 30 |
| *Candida curvata R* (IOWA) | 33 |
| *Candida curvata D* (IOWA) | 35 |
| *Candida 107* | 25 |

In the above NCYC indicates microbes of the National Collection of Yeast Cultures.

The typewriter test gave very clear and effective copies similar to that obtained in Example I with all of these microbes.

Example III

The procedure of Example I was repeated except that the following liquids were employed individually in place of the 2-ethylhexyl acetate, viz. octanol, decanol, a commercially available mixture of C12 and C13 primary alcohols, a commercially available mixture of C14 and C15 primary alcohols, a commercially available mixture of C9 to C11 primary alcohols, diethylene glycol (digol), di-iso-butyl phthalate, butyl benzyl phthalate, acetyl tributyl citrate, 2,2,4-trimethyl-1,3-pentanediol iso-butyrate, glyceryl triacetate (triacetin) and glyceryl tributyrate.

All of these liquids resulted in a satisfactory "carbonless," copy paper.

Example IV

The procedure of Example I was repeated except that a 2% (w/v) solution of crystal violet lactone in di-2-ethylhexyl adipate ("Lankroflex" DOA) was employed in place of the 1% 2-ethylhexyl acetate solution.

A satisfactory "carbonless" copy paper was produced.

Example V

The procedure of Example I was repeated except that the time for which the aqueous microbial slurry was stirred with the solution of crystal violet lactone in 2-ethylhexyl acetate was varied. The times were 1, 2, 3, 4, 6 and 24 hours.

The typewriter test showed that the product of the 3-hour incubation time was as good as the products of the higher incubation times.

Example VI

The procedure of Example I was repeated except that the concentration of crystal violet lactone in the 2-ethylhexyl acetate was 3% (w/v) and the aqueous microbial slurry was stirred with the dye/acetate solution for 3 hours at 60°C.

The typewriter test gave improved copies compared to that of Example I.

Example VII

The procedure of Example VI was repeated except that the following quantities of dye/acetate solution were employed individually, viz 1.0, 0.8, 0.6 and 0.4 g per g dry microbe.

The typewriter copy produced by the employment of the 0.6 quantity was similar to the copies produced by employing the 0.8 and 1.0 quantities and was superior to that produced by employing the 0.4 quantity.

Example VIII

*Saccharomyces cerevisiae* and *Candida utilis* 707 were individually cultivated under reduced nitrogen conditions and harvested, by the procedure of Example I.

1 g of each strain was made into a 15% aqueous slurry and then mixed with 1 g of a 1% (w/v) solution of crystal violet lactone in 2-ethylhexyl acetate. Each mixture was stirred as described in Example I for 3 hours and the resulting encapsulated dye was harvested by centrifugation.

The capsules were applied to separate sheets of paper and subjected to a typewriter test as described in Example I. Both products gave a satisfactory print.

The total lipid contents (% by weight based on the dry weight of the microbe), measured as described in Example I, were 11.3% for the *S. cerevisiae* and 12.3% for the *C. utilis* 707.

Example IX

A culture of *Lipomyces starkeyi* 1809 was grown in the following medium:

|  | g.l$^{-1}$ |
|---|---|
| sucrose | 40 |
| ammonium tartrate | 1.5 |
| potassium dihydrogen orthophosphate | 5.0 |
| magnesium sulphate 7H$_2$O | 0.2 |
| sodium chloride | 0.1 |
| calcium chloride 2H$_2$O | 0.01 |
| yeast extract powder | 1.0 |

The culture was grown in 10-litre fermentation vessel at 28°C, using a stirrer speed of 200 rpm and an aeration rate of 1 volume/volume/minute. The grown yeast cells were harvested by centrifugation at 2000 rpm for 10 minutes.

The total lipid content of the yeast, determined using the same procedure as in Example I, was found to be 35.12% by weight based on the dry weight of the yeast.

A 20% aqueous slurry containing 1 g (dry weight) of the microbe was mixed with 0.8 g of a 2.5% w/w solution of crystal violet lactone known as Pergascript Blue I—2R and benzoyl leuco methylene blue known as Pergascript Blue S—4G (1.5:0.5 g mixture) in octan-2-ol. The mixture was stirred for 2½ hours at 50°C using a laboratory hot plate magnetic stirrer at a medium speed to ensure homogeneity of the mixture.

The resulting microbial encapsulated dye was harvested by centrifugation at 2000 rpm for 10 minutes. The harvested capsules were suspended in distilled water at a concentration of approximately 25% dry weight of capsules in water, and the suspension was applied to the opposite side of clay-coated paper as described in Example I. The quantity of capsules applied to the paper was of the order of 3—6 g.m⁻². The paper was dried and tested for duplication ability using a standard office typewriter. The copies produced were satisfactory.

Example X

The procedure of Example IX was followed except that xylene was employed in place of the octan-2-ol. The typewriter test gave a satisfactory copy.

Example XI

The procedure of Example IX was followed except that a 0.7 g of a 2% w/w solution of the dye mixture in a 50/50 w/w blend of acetone (as thinner) and a commercially available mixture of C12 to C15 long-chain primary alcohols was employed in place of the octan-2-ol solution.

The typewriter test gave improved copies compared to when the C12 to C15 alcohol mixture and acetone were employed individually.

Example XII

The procedure of Example IX was followed except that "HB 40" (a commercially available mixture of 40% hydrogenated terphenyls) was employed in place of the octan-2-ol and the mixing time was 3 hours.

The typewriter test have a positive print but not as good as that produced by Example I.

This procedure was repeated except that a 50/50 w/w blend of acetone and "HB 40" was employed in place of the "HB 40".

The typewriter test gave a better copy than when "HB 40" alone was employed.

Example XIII

The procedure of Example XII was repeated except that "Santosol 340" (a commercially available mixture of hydrogenated aromatic hydrocarbons) was employed in place of the "HB 40".

The typewriter test gave similar results as for Example XII, the acetone/"Santosol" blend giving better copies than "Santosol" alone.

Example XIV

A 20% aqueous slurry containing 1 g (dry weight) of the microbe produced according to Example IX was mixed with 0.8 g of 2-ethylhexyl acetate by medium stirring using a laboratory hot plate magnetic stirrer for 1½ hours at 50°C and the resulting lipid-extended microbe was harvested by centrifugation. Microscopic examination showed a large glistening globule occupying the whole of the cytoplasm of the yeast cell.

A 20% aqueous slurry containing 1 g (dry weight) of the lipid-extended microbe was mixed with 0.8 g of a 2% (w/v) solution of Leucodye in propanol (immiscible with soybean oil but miscible with 2-ethylhexyl acetate) by medium stirring using a laboratory hot plate magnetic stirrer for 2½ hours at 50°C and the resulting microbe was harvested by centrifugation.

The typewriter test gave a satisfactory copy.

A parallel procedure was carried out except that the microbe was not treated with 2-ethylhexyl acetate. The typewriter test gave a poorer copy than when 2-ethylhexyl acetate was employed.

Example XV

A 20% aqueous slurry containing 3 g (dry weight) of the yeast prepared as described in Example I was mixed with 3 g of clove oil and 0.5 ml of 2-ethylhexyl acetate by medium stirring using a laboratory hot plate magnetic stirrer for 3 hours at 50°C. The microbial product was harvested by centrifugation and oven-dried at 70°C.

When the resulting microbe was crushed, a distinct odour of clove oil was noticed.

**Claims**

1. A method of producing an encapsulated material which comprises treating a grown microbe with a material to be encapsulated, characterised in that the microbe is contacted with an organic lipid-extending substance being an organic liquid which is capable of being taken up by the microbe so that it is retained passively therein and which is a solvent or microdispersing medium for the material to be encapsulated, and simultaneously and/or subsequently contacting the microbe with the material to be encapsulated, said material being employed as a solution or microdispersion in the organic lipid-extending substance or as a solution or microdispersion in a further organic lipid-extending substance or in a liquid which is miscible with the first-mentioned lipid-extending substance, so that both the organic lipid-extending substance and the material are taken into and retained passively in the microbe.

2. A method according to claim 1 characterised in that the microbe is a yeast.

3. A method according to claim 1 or 2 characterised in that the substance is a liquid selected from aliphatic alcohols, esters, aromatic hydrocarbons and hydrogenated aromatic hydrocarbons, provided that the selected liquid is capable of being taken up by the microbe and retained passively therein and provided that the material to be encapsulated is soluble or microdispersible in the selected liquid.

4. A method according to claim 3 characterised in that the substance is selected from primary alcohols having 4 to 15 carbon atoms, isobutanol, octan-2-ol, tertiary-butanol, diethylene glycol, di-2-ethylhexy adipate, di-iso-butyl phthalate, butyl benzyl phthalate, acetyl tributyl citrate, 2,2,4 - trimethyl - 1,3 - pentanediol isobutyrate, glyceryl triacetate, glyceryl tributyrate, 2-ethylhexyl acetate, and xylene.

5. A method according to any of the preceding claims characterised in that the substance is diluted by blending with a thinner such as acetone.

6. A method according to any of the preceding claims characterised in that the microbe is in the form of an aqueous slurry for mixing with the substance.

7. A method according to any of the preceding claims characterised in that the microbe has a lipid content of up to 35% by weight.

8. A method according to any of the preceding claims characterised in that the material is a leuco dye suitable for use in carbonless copy paper.

9. A method according to any of claims 1 to 7 characterised in that the material is an insecticide.

10. Carbonless copy paper employing as the encapsulated dye a microbially encapsulated dye produced by a method according to any of claims 1 to 8 when employing a dye which is dissolved or microdispersed in the substance.

**Patentansprüche**

1. Verfahren zum Erzeugen eines eingekapselten Materials, bestehend aus der Behandlung einer gewachsenen Mikrobe mit einem einzukapselnden Material, dadurch gekennzeichnet, daß die Mikrobe mit einer organischen lipoid-streckenden Substanz in Berührung gebracht wird, welche eine organische Flüssigkeit ist, die zur Aufnahme durch die Mikrobe befähigt ist, so daß sie darin passiv zurückgehalten wird, und die ein Lösungs- oder Mikrodispersions-Mittel für das einzukapselnde Material ist, und gleichzeitig und/oder darauffolgend die Mikrobe mit dem einzukapselden Material in Berührung gebracht wird, wobei das Material als eine Lösung oder Mikrodispersion in der organischen lipoid-streckenden Substanz oder als eine Lösung oder Mikrodispersion in einer weiteren organischen lipoid-streckenden Substanz oder in einer Flüssigkeit benutzt wird, welche mit der ersterwähnten lipoid-streckenden Substanz mischbar ist, so daß sowohl die organische lipoid-streckende Substanz als auch das. Material in die Mikrobe aufgenommen und passiv in ihr zurückgehalten werden.

2. Verfahren nach Anspruch 1, dadurch gekennzeichnet, daß die Mikrobe eine Hefe ist.

3. Verfahren nach Anspruch 1 oder 2, dadurch gekennzeichnet, daß die Substanz eine Flüssigkeit ist, ausgewählt aus aliphatischen Alkoholen, Estern, aromatischen Kohlenwasserstoffen und hydrierten aromatischen Kohlenwasserstoffen, vorausgesetzt, daß die ausgewählte Flüssigkeit befähigt ist, von der Mikrobe aufgenommen und passiv in ihr zurückgehalten zu werden, und vorausgesetzt, daß das einzukapselnde Material in der ausgewählten Flüssigkeit löslich oder mikrodispersionsfähig ist.

4. Verfahren nach Anspruch 3, dadurch gekennzeichnet, daß die Substanz ausgewählt wird aus primären Alkoholen mit vier bis fünfzehn Kohlenstoffatomen, Iso-Butanol, Octan-2-ol, Tertiär-Butanol, Diethylen-Glycol, Di-2-Ethylhexyl-Adipat, Di-Iso-Butyl-Phthalat, Butyl-Benzyl-Phtalat, Acetyl-Tributyl-Zitrat, 2,2, 4 - Trimethyl - 1 - 3 - Pentandiol - Iso - Butyrat, Glyceryl-Triacetat, Glyceryl-Tributyrat, 2-Ethylhexyl-Acetat und Xylol.

5. Verfahren nach einem der vorangehenden Ansprüche, dadurch gekennzeichnet, daß die Substanz durch Mischen mit einem Verdünnungsmittel wie Aceton verdünnt wird.

6. Verfahren nach einem der vorangehenden Ansprüche, dadurch gekennzeichnet, daß die Mikrobe in Form eines wässrigen Schlammes zum Mischen mit der Substanz vorliegt.

7. Verfahren nach einem der vorangehenden Ansprüche, dadurch gekennzeichnet, daß die Mikrobe einen Lipoid-Gehalt von bis zu 35 Gewichtsprozent besitzt.

8. Verfahren nach einem der vorangehenden Ansprüche, dadurch gekennzeichnet, daß das Material ein zur Verwendung bei carbonfreiem Durchschreibepapier geeigneter Leuko-Farbstoff ist.

9. Verfahren nach einem der Ansprüche 1 bis 7, dadurch gekennzeichnet, daß das Material ein Insektizid ist.

10. Carbonfreies Durchschreibepapier, bei dem als eingekapselter Farbstoff eine mikrobisch eingekapselte Farbe verwendet wird, die durch ein Verfahren nach einem der Ansprüche 1 bis 8 erzeugt ist unter Verwendung eines Farbstoffes, der in der Substanz aufgelöst oder als Mikrodispersion enthalten ist.

**Revendications**

1. Procédé de production d'une matière encapsulée comprenant le traitement d'un micro-organisme développé avec la matière à encapsuler, procédé caractérisé en ce que l'on met le micro-organisme en contact avec une substance organique diluant les lipides qui est un liquide organique pouvant être absorbé par le micro-organisme et y être ainsi retenu passivement, et qui est un solvant ou un milieu de microdispersion pour la matière à encapsuler, et, simultanement et/ou ensuite, on met le micro-organisme en contact avec la matière à encapsuler, cette matière étant employée en solution ou en micro-dispersion dans la substance organique diluant les lipides, ou bien en solution ou en microdispersion dans une autre substance organique diluant les lipides ou dans un liquide miscible avec la première substance indiquée, à le fois la substance diluant les lipides et la matière étant ainsi prise et retenue passivement dans le micro-organisme.

2. Procédé selon la revendication 1, caractérisé en ce que le micro-organisme est une levure.

3. Procédé selon la revendication 1 ou 2, caractérisé en ce que la substance est un liquide choisi parmi des alcools aliphatiques, ester et hydrocarbures aromatiques éventuellement hydrogénés, avec les conditions que ce liquide puisse être absorbé par le micro-organisme et y être retenu passivement, et que la matière à encapsuler soit soluble ou microdispersable dans le liquide choisi.

4. Procédé selon la revendication 3, caractérisé en ce que la substance est choisie parmi des alcools primaires en $C_4$ à $C_{15}$, l'isobutanol, l'octane-2-ol, le butanol tertiaire, le diéthylène-glycol, l'adipate de di-éthyl-2-hexyle, le phtalate de di-isobutyle, le phtalate de butyle et benzyle, le citrate d'acétyle et tributyle, l'iso-butyrate de triméthyl - 2,2,4 - pentane diol 1,3, le triacétate de glycéryle, le tributyrate de glycéryle, l'acétate d'éthyl-2 hexyle, et le xylène.

5. Procédé selon l'une quelconque des revendications 1 à 4, caractérisé en ce que la substance est diluée par mélange avec un diluant tel que l'acétone.

6. Procédé selon l'une quelconque des revendications précédentes, caractérisé en ce que le micro-organisme est en suspension aqueuse pour le mélange avec la substance.

7. Procédé selon l'une quelconque des revendications précédentes, caractérisé en ce que le micro-organisme a une teneur en lipides pouvant s'élever jusqu'à 35% en poids.

8. Procédé selon l'une quelconque des revendications précédentes, caractérisé en ce que la matière est un leuco-colorant propre à être employé dans un papier de copie sans carbone.

9. Procédé selon l'une quelconque des revendications 1 à 7, caractérisé en ce que la matière est un insecticide.

10. Papier de copie sans carbone dans lequel on utilise comme colorant encapsulé un colorant encapsulé par voie microbienne qui a été obtenu par un procédé selon l'une quelconque des revendications 1 à 8 quand on emploit un colorant qui est dissous ou microdispersé dans la substance.